# EUROPEAN PATENT APPLICATION

(11) **EP 4 088 679 A1**
(43) Date of publication of application: **16.11.2022**
(21) Application number: 21305608.8
(22) Date of filing: 11.05.2021
(51) Int. Cl.: A61B 34/20, A61B 34/30, A61B 34/10, A61B 17/00, A61B 90/00

(54) **SYSTEM FOR IDENTIFYING AN UNEXPECTED MOTION OF A TRACKER ATTACHED TO A PATIENT DURING SURGERY**

(71) Applicant: Ecential Robotics, 38610 Gieres (FR)
(72) Inventor: LAVALLEE, Stéphane, 38410 Saint-Martin d'Uriage (FR); ARMAND, David, 38120 Saint-Égrève (FR); VIDAL, Clément, 38240 Meylan (FR); CHABANAS, Laurence, 38830 Crêts-en-Belledonne (FR)
(74) Representative: Regimbeau

(57) **Abstract**

The invention relates to a system (100) for identifying an unexpected motion of a patient tracker (10) attached to a patient (2) during surgery, the system (100) comprising
a. a patient tracker (10)
b. a localization system (103) coupled to the patient tracker (10),
c. a memory (102) in which a set of expected motions of the patient tracker is recorded,
d. a control unit (101) connected to the memory (102) and the localization system (103), the control unit (101) being configured to monitor a motion of the patient tracker (10) during surgery, determine a difference between the motion of the patient tracker (10) and the set of expected motions of the patient tracker (10), and compare the difference to a predetermined threshold,
e. an alert module (105) connected to the control unit (101), the alert module (101) being configured to emit an alert of an unexpected motion, when the difference exceeds the predetermined threshold.

## Description

### FIELD OF THE INVENTION

The present invention relates to the field of systems for identifying motions of a tracker attached to a patient during surgery, and especially unexpected motions of a patient during surgery.

### BACKGROUND OF THE INVENTION

In surgery, X-ray imaging is frequently employed to guide surgical procedures. In particular in orthopedic, spine and traumatology surgery, X-ray imaging is frequently employed to monitor fracture reduction, to insert instrument at precise locations and to position implants in one or several bone fragments. X-ray imaging can produce a 2D image or a 3D image reconstructed from multiple 2D images using tomography reconstruction algorithms.

Guiding surgical procedures requires the instruments used during the surgical intervention to be equipped with a tracking assembly or tracker. For instance, a tracking assembly of optical, electromagnetical, ultrasonic or inertial type may be used. The tracking assembly is coupled to a localization system.

Another tracker is mounted onto the patient and is also coupled to the localization system.

The whole relevancy and safety of guided surgical procedures lies on the stability and rigidity of the geometrical relationship between each tracker and the item it is supposed to track.

However during surgery this relationship may be modified, especially for an instability of the tracking assembly mounted onto the patient due to an excessive force applied on the tracker or a dysfunctional fixation of the tracker.

The surgical operation cannot be carried on as the safety and accuracy of the guided surgical procedure is no longer guaranteed.

In such case, the surgical operation is put on hold to calibrate again the geometrical relationship between the failing tracking assembly and its corresponding item. It is a time-consuming process uncomfortable for the surgeon.

A solution to generate an alert could consist in attaching two different and independent tracking assemblies onto the patient. In such case, a change in the relative position of the trackers reveals a displacement of one of the trackers with respect to its nominal position. An alert may then be sent to the surgeon to stop the operation and launch a recalibration process.

However, a change in the relative position of the tracking assemblies may also be unharmful as the patient experiences natural and expected motions during surgery, in particular due to breathing. Emitting an alert for any change in the relative position of the trackers would lead to useless and time-consuming tasks.

There is a need to provide a method for identifying an unexpected motion of a tracking assembly attached to a patient.

### BRIEF DESCRIPTION OF THE INVENTION

It is an object of this invention to provide a method for identifying an unexpected motion of a tracker attached to a patient that allows the generation of a meaningful alert when the tracker gets at least partially detached from the patient so that the safety and accuracy of the navigation system is no longer guaranteed.

To that end, the invention provides a system for identifying an unexpected motion of a patient tracker attached to a patient during surgery, the system comprising
a. a patient tracker
b. a localization system coupled to the patient tracker,
c. a memory in which a set of expected motions of the patient tracker is recorded,
d. a control unit connected to the memory and the localization system, the control unit being configured to
   monitor a motion of the patient tracker during surgery, determine a difference between the motion of the patient tracker and the set of expected motions of the patient tracker, and
   compare the difference to a predetermined threshold,
e. an alert module connected to the control unit, the alert module being configured to emit an alert of an unexpected motion, when the difference exceeds the predetermined threshold.

This system involves a predetermined threshold and a set of expected motions so that an alert is emitted only if the actual motion of the tracker differs significatively from its expected motion. The setting of the threshold allows to avoid the emission of useless alerts when the motion of the tracker is due to expected motions of the patient during surgery.

Such system is advantageously and optionally completed by the following features taken singly or in combination:
a user interface configured to
   display a proposition to the user to add the unexpected motion to the set of expected motions and
   receive a refusal or an approval from the user,
the control unit being configured to add the unexpected motion to the set of expected motions when an approval is received from the user;
the set of expected motions of the bone is produced based on a model;
the control unit is further configured to modify the model so as to consider the unexpected motion as an expected motion, when an approval is received from the user;
the model includes a breathing model of the patient;
the control unit is configured to record a reference motion of the patient tracker during a breathing cycle intraoperatively, and add the reference motion to the set of expected motions;
the control unit is configured to receive a position and an orientation of a navigated instrument, the control unit being configured to determine the set of expected motions in function of the position and the orientation of the navigated instrument;
the control unit is configured to receive at least one of the following parameters of the instrument: an entry point in the bone, an entry angle and a velocity, the control unit being configured to determine the set of expected motions in function of said at least one parameter;
the control unit is configured to modify the predetermined threshold depending on a distance between a tip of the navigated instrument and a surgical target;
the control unit is configured to increase the predetermined threshold when the distance decreases;
the system further comprises a surgical robotic system configured to guide the instrument;
the system further comprises a surgical robotic system configured to autonomously actuate the instrument;
the control unit is configured to define a reference coordinate system fixed with respect to an operating room, the control unit being configured to express the motion of the patient tracker and the set of expected motions of the patient tracker in the reference coordinate system;
the control unit is configured to receive from the localization system localization data relating to a position and an orientation of a reference tracker located on a base of the surgical robotic system, the base being fixed with respect to the operating room, the control unit being configured to calculate a motion of the patient tracker with respect to the position and orientation of the reference tracker;
the control unit is configured to receive from the localization system localization data relating to a position and an orientation of a reference tracker located on an end-effector of a robotic arm of the surgical robotic system, the control unit being configured to calculate a position and orientation of the reference coordinate system based on a geometrical model of the robotic arm and calculate a motion of the patient tracker with respect to the reference coordinate system.

### BRIEF DESCRIPTION OF THE DRAWINGS

Further features and advantages of the invention will be apparent from the following description, based on the appended drawings wherein:
Fig. 1 represents schematically a system for identifying an unexpected motion of a tracker according to an embodiment of the invention;
Fig. 2 represents schematically a tracking assembly or tracker and a registration phantom attached to a bone of a patient;
Fig. 3 represents schematically a tracking assembly or tracker attached to a bone of a patient2;
Fig. 4 represents schematically a robotic surgery system;
Fig. 5 represents schematically a virtual trajectory to place a pedicle screw placement.
Fig. 6 represents schematically a system for identifying an unexpected motion of a tracker according to an embodiment of the invention;
Fig. 7 represents schematically an expected displacement of a tracker attached to a bone of a patient due to breathing;
Fig. 8 represents schematically an expected displacement of a tracker attached to a bone of a patient due to an interaction of a surgical instrument with said bone.

Reference signs that are identical from one figure to another one designate the same element, or elements fulfilling the same function.

### DETAILED DESCRIPTION OF EMBODIMENTS OF THE INVENTION

### System for identifying an unexpected motion of a tracker

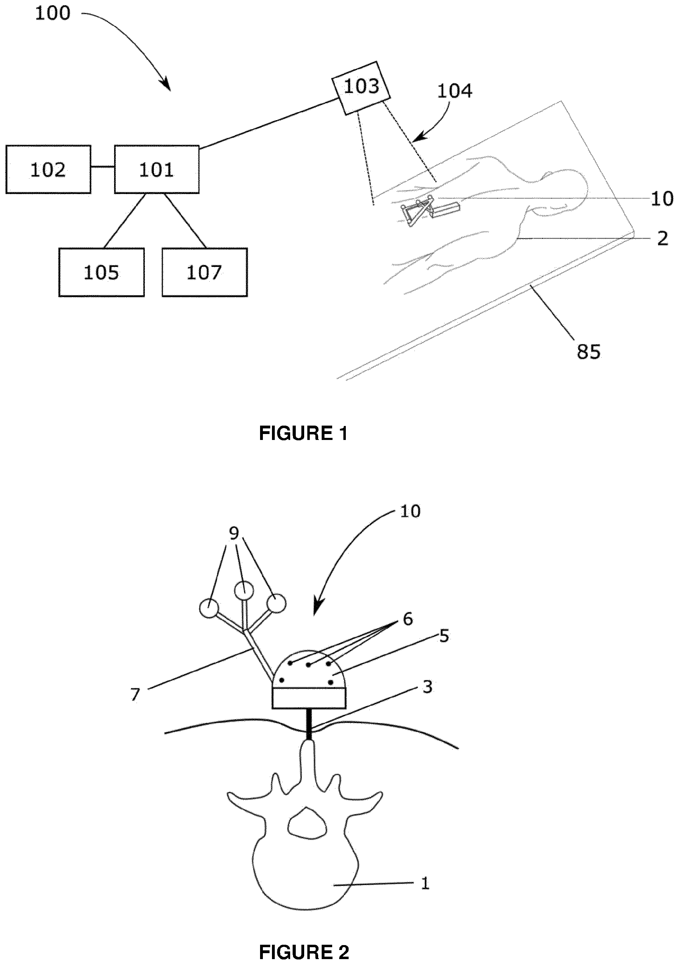

Figure 1 illustrates a system 100 for identifying an unexpected motion of a tracking assembly or tracker 10.

A patient 2 lies on an operating table 85.

The system 100 comprises a tracker 10 attached to the patient 2. The tracker 10 attached to the patient is further designated as the patient tracker 10.

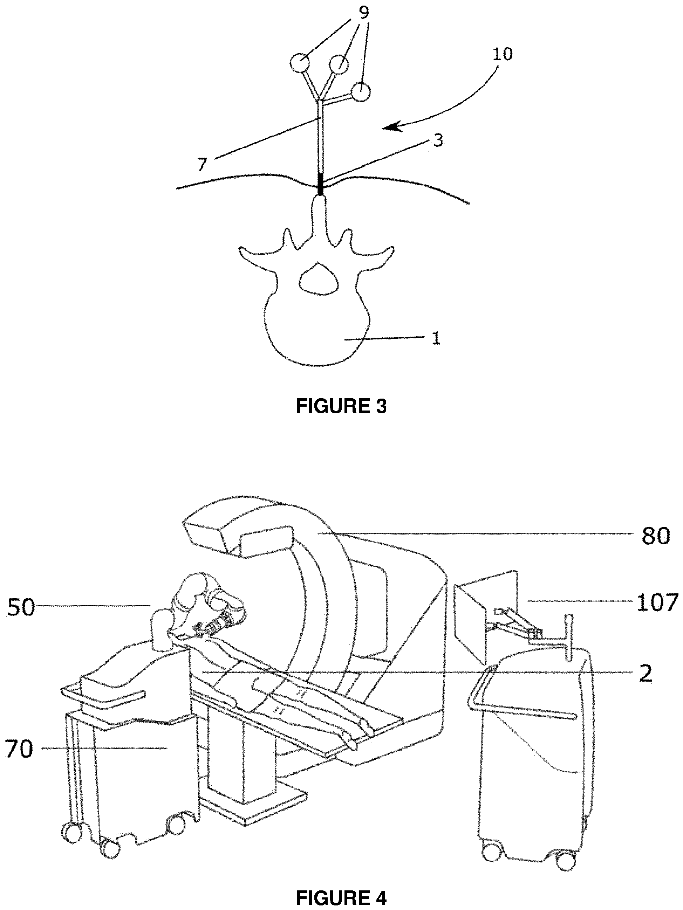

Figures 2 and 3 illustrate a tracking assembly or tracker 10 attached to a bone 1 of the patient 2. For example, the bone 1 may be a vertebra.

The tracker 10 comprises an attachment 3 that rigidly links the tracker 10 to the bone 1. In the illustrated embodiment, the attachment is represented as a pin implanted into the bone. However, in alternative embodiments (not illustrated), the attachment may be non-invasive, such as an adhesive placed onto the patient's skin in the vicinity of the bone.

The tracker 10 comprises a plurality of markers, the shape of the markers and the arrangement of the plurality of markers being predetermined and characterized before using the tracker 10.

In the illustrated embodiment of figures 2 and 3, the tracker 10 comprises a rod 7 supporting at least three markers 9 having a known shape and size (e.g. balls or disks) arranged in a known position.

As illustrated in figure 2, the tracker 10 may also comprise a registration phantom 5 made of a radiotransparent material. The registration phantom 5 comprises a plurality of radiopaque fiducials 6 having a known shape and size (e.g. balls or pins) arranged in a known position.

Both tracker 10 and registration phantom 5 are rigidly attached to the attachment 3 via a common base, and are thus rigidly attached to the bone 1.

The geometrical relationship between the tracker 10 and the bone 1 is determined so that measuring the position and orientation of the tracker 10 enables to determine the position and orientation of the bone 1.

The system 100 further comprises a localization system 103.

The tracker 10 is coupled to the localization system 103 to measure the position and orientation of the tracker. The tracker 10 is coupled to the localization system 103 when the localisation system 103 is configured to detect the tracker and locate it in space.

The localization system may be of the optical type, such as a camera and particularly an infrared camera.

Figure 1 illustrates a localization system 103 of the optical type configured to detect the position and orientation of the patient tracker 10 only if the patient tracker 10 is located within a cone of vision 104. However, the invention is not limited to this type of localization system.

The system 100 further comprises a memory 102 in which a set of expected motions of the tracker is recorded.

The system 100 comprises a control unit 101 configured to monitor a motion of the tracker during surgery, determine a difference between the motion of the tracker and the set of expected motions of the tracker, and compare the difference to a predetermined threshold.

In order to determine the difference, the motion of the tracker and the set of expected motions of the tracker are expressed in a reference coordinate system.

The reference coordinate system will be explained in more detail further in the text.

The control unit 101 is connected to the memory 102 in order to access the set of expected motions.

The control unit 101 is also connected to the localization system 103 in order to access the motion of the tracker during surgery.

The system 100 comprises an alert module 105 configured to emit an alert of an unexpected motion, when the difference exceeds the predetermined threshold.

The alert module 105 may be a sound emitter, a light emitter or a screen. The alert may be a sound, a light or a message displayed on a screen.

Optionally, the control unit 101 is connected to a user interface 107 in order to display information to a user and receive information from a user, for instance a surgeon. The user interface 107 is possibly comprised in the control unit 101.

The interface 107 may emit an alert of an unexpected motion and replace or include the alert module 105 for that purpose.

When an alert of an unexpected motion is emitted by the alert module 105 and/or the user interface 107, the user interface may optionally display a proposition to the user to add the unexpected motion to the set of expected motions. The user may give his refusal or approval to this proposition through the interface 107.

The control unit adds the unexpected motion to the set of expected motions when an approval is received from the user.

When a refusal is received from the user, the set of expected motions remains unchanged.

In this way, detected unexpected motion and corresponding alarms due to an excessive force applied on the tracker or a dysfunctional fixation of the tracker can be addressed by the user, e.g. by checking the attachment of the trackers and, if appropriate, correcting a dysfunctional attachment of the trackers before carrying on the surgical procedure.

As will be explained in more detail below, the set of expected motions of the bone may be produced based on a model.

In the situation where the user discards an alert via the user interface 107, the movement initially classified as unexpected and at the origin of the alert may be used to enrich and enhance the model producing the expected movements.

To this end, the interface may also propose to the user to qualify said movement for further processing and integration into the corresponding model.

For example, if the movement initially classified as unexpected turns out to come from an interaction between an instrument manipulated by the surgeon and the bone, the surgeon may indicate, through the user interface, that the movement must be added to a bone/instrument mechanical interaction model. The control unit records the configuration corresponding to the bone and the instrument the navigation data at the time of the alert and update a bone/instrument mechanical interaction model so that the similar values of those parameters do not trigger an alert. As a result, if a similar interaction occurs subsequently during the very same surgical operation or during another prospective surgical operation, the control unit will not generate any alert, in order not to disturb the user.

Optionally, the control unit 101 is configured to record a monitored motion of the tracker during surgery, and add it to the set of expected motions. The monitored motion may be acquired by the localization system 103 and recorded in the memory 102. For instance a motion of the patient tracker during one or several breathing cycle(s) may be acquired and recorded intraoperatively.

The expression « intraoperatively » designates in this text the period of time during which the patient lies on the operating table and the surgeon may begin the surgery operation or the surgeon has started the surgery operation.

The expression « during surgery » designates in this text the period of time during which the patient lies on the operating table and the surgeon performs the surgical operation.

### Navigation of a surgical instrument

During surgery, the instrument used to carry out the surgical procedure is navigated with respect to a 2D or 3D image of the patient's bone.

An X-ray imaging system is employed to produce 2D images or a 3D image reconstructed from multiple 2D images using tomography reconstruction algorithms, such as CBCT (Cone Beam Computed Tomography), using the registration phantom mentioned above.

Such images may be taken intraoperatively, in particular before the surgeon begins the surgical operation itself.

The images are used to define a virtual trajectory that the instrument needs to follow to perform the surgical operation. This virtual trajectory defines a planning that may be recorded in the memory 102 of the control unit 101.

Since the registration phantom is fixed on the same base as the patient tracker, the position of the virtual trajectory relative to the patient tracker may be determined.

Specific parameters may be extracted from the planning such as the position of a bone that is supposed to be affected during surgery, the position of an entry point where the instrument is supposed to come into contact with the bone during surgery, or an entry axis or entry direction with which an extension of the instrument is supposed to be aligned during surgery. Those parameters may constitute surgical targets possibly recorded in the memory of the control unit 101.

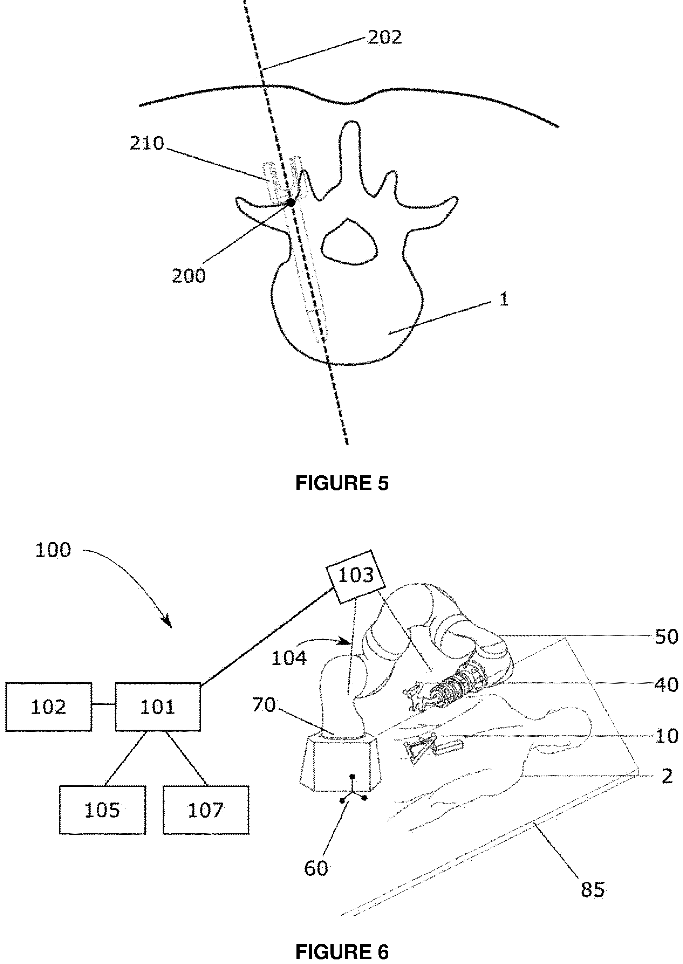

For example, figure 5 illustrates the case of a pedicle screw placement. A virtual trajectory 202 defines the successive positions that an instrument is supposed to follow so to drill the bone 1. This hole is configured to eventually receive the pedicle screw 210. The intersection point of the virtual trajectory 202 with the bone 1 defines the entry point 200. An entry point of an instrument acting on a bone is thus a point located on the surface of the bone, the instrument entering or coming into contact the bone at the entry point.

The instrument may be navigated by fixing a tracker, called instrument tracker, to the instrument and coupling the localization system to the tracker. In this way, the position and orientation of the tracker relative to the virtual trajectory may be determined in real time.

### Surgical robotic system

The system presented here above may be used in collaboration with a robotic surgical system 70 as illustrated in figure 4.

Surgical robotic systems are frequently used during surgical interventions, in order to assist a surgeon. The surgical robotic system may guide the positioning and orientating of a surgical instrument for the surgeon.

The robotic surgical system 70 comprises a surgical robotic arm 50 located close to patient 2.

The distal end of the robotic arm 50 comprises an end effector. The end effector may either hold a tool guide configured to guide the instrument, or the instrument itself.

The robotic arm is controlled to place the tool guide or the instrument with a given position and orientation in order to reach a surgical target. A surgical target can be a specific position to be reached by the instrument, or a specific orientation to be reached by an instrument or a combination of a position and an orientation.

The tool guide may be used to provide an intuitive indication of an axis or a position to the surgeon. In that case, the instrument is directly and freely manipulated by the surgeon through the tool guide. Once the tool guide is correctly placed, the surgeon can insert the instrument into the tool guide, so as to maneuver the surgical tool according to the correct position and/or orientation in order to proceed with the surgery.

Alternatively, if the instrument is held by the end effector, the robotic arm may fully and automatically move the instrument. The instrument is then moved by the surgical robotic system, meaning that the position and orientation of the instrument is set and modified automatically by the robotic system. In this situation, the instrument may optionally be autonomously actuated by the surgical robotic system, meaning that an active function of the instrument, such as a drilling function, is launched and stopped automatically by the robotic system.

A first way to monitor the instrument consists in rigidly fixing a tracker, called instrument tracker, to the instrument and coupling the localization system to the tracker.

A second way to monitor the instrument may be implemented when the instrument is guided or held by the surgical robotic system, a tracker being attached to the end effector or to another part of the robotic arm. The position and orientation of the instrument may then be deduced from the position and orientation of the tool guide.

The control unit 101 is connected to the surgical robotic system to receive in real-time the position and orientation of the instrument or the end effector.

Figure 6 illustrates a system 100 for identifying an unexpected motion of a tracking assembly or tracker 10, in the case where a robotic surgery system 70 is also involved in the surgery operation. An X-ray imaging system 80 is also involved. An instrument tracker 40 is rigidly fixed to the tool guide and the localization system 103 may be used to locate the tool guide tracker.

In the case where a navigated instrument is involved, the predetermined threshold to which the difference between the motion of the patient tracker 10 and the set of expected motions of the patient tracker 10 is compared, may optionally depend on the distance between a tip of the navigated instrument and a surgical target. More specifically, the predetermined threshold may be increased as the distance decreases. If the instrument is guided by the surgical robotic system and does not carry any tracker, the position of the tip may be determined by placing the instrument in abutment with the tool guide and computing the position of the tip based on the position of the tool guide and the length of the instrument.

As the control unit 101 receives in real-time navigation information of the instrument and position and orientation of the bone, the control unit 101 updates the value of the predetermined threshold in real-time.

Such a threshold adapted in function of the proximity of the instrument to the bone enables to avoid alerting the user too often during a normal procedure.

On the opposite, if no instrument is located in the vicinity of the entry point, and more generally in the vicinity of the surgical area, the threshold may be lower because apart from the breathing motion, no other motion of the tracking assembly is expected.

### Reference coordinate system and target reference system

The reference coordinate system is a coordinate system in which motions of the tracker during surgery are expressed.

Other relevant motions may be monitored and expressed in the reference coordinate system, such as the motion of a second surgical target such as a second bone, or the motion of a surgical instrument that is supposed to act on a surgical target.

The reference system may be a fixed coordinate system in the operating room, and may be defined relative to the operating room or a fixed object in the operating room. For example, the reference system may be defined relatively to a fixed base of the surgical robotic system, if such a surgical robotic system is used in the surgical procedure and fixed within the operating room. Alternatively, the reference coordinate system may be associated to a reference tracker rigidly fixed to the operating table, if the operating table remains fixed in the operating room.

For instance, as illustrated in figure 6, the control unit 101 may be configured to receive from the localization system localization data relating to the position and orientation of a reference tracker 60 located on the base of the surgical robotic system, the base being fixed with respect to the operating room, the control unit being configured to calculate a motion of the patient tracker with respect to the position and orientation of the reference tracker.

Alternatively, the reference coordinate system may be defined relatively to a moving object within the operating room. For example, the reference system may be defined relative to an end-effector located on a distal end of a robotic arm of a surgical robotic system. In this option, the motion of the end-effector relative to the operating room or a fixed object in the operating room is monitored at all times during surgery. The reference coordinate system may be associated to a reference tracker rigidly fixed to the moving object and coupled to the localization system.

For instance, the control unit may be configured to receive from the localization system localization data relating to the position and orientation of a reference tracker 40 located on an end-effector of a robotic arm of the surgical robotic system, the control unit being configured to calculate a position and orientation of the reference coordinate system based on a geometrical model of the robotic arm and calculate a motion of the patient tracker with respect to the reference coordinate system.

The patient tracker 10 linked to the bone 1 defines a target coordinate system. The target coordinate system may be in translation and/or rotation with respect to the reference coordinate system. Monitoring the tracker linked to the bone 1 during surgery enables to follow this translation and rotation.

### Expected Motions

A first example of expected motions of the patient during surgery is the natural or artificial breathing of the patient.

The bone 1 may experience breathing-induced motions, dragging the bone tracker in the breathing-induced motion.

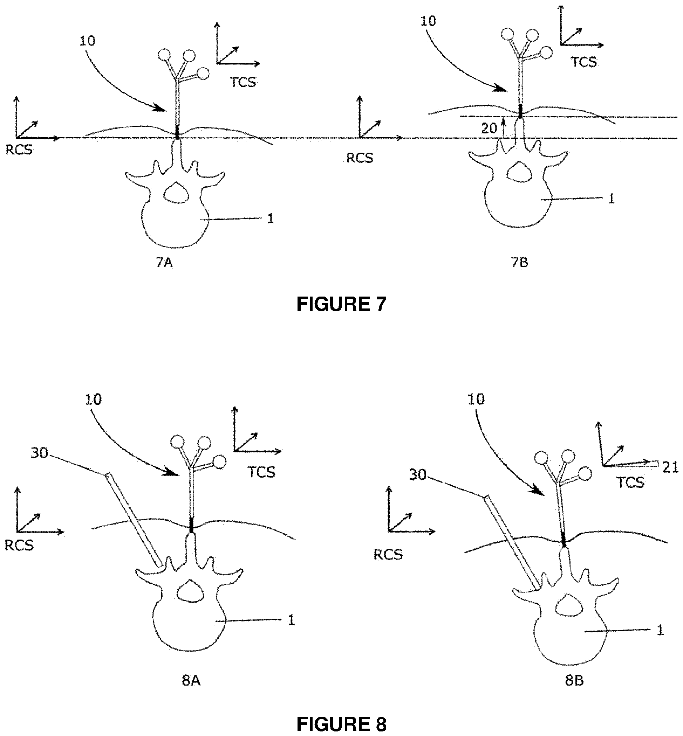

Parts 7A and 7B of figure 7 illustrate such a breathing-induced motion, each part illustrating the bone 1 and the tracker 10 at two different instants of a breathing cycle.

Both parts comprise the reference coordinate system RCS that remains motionless with respect to the breathing.

Both parts comprise a target coordinate system TCS virtually attached to the tracker linked to the bone and thus put in motion by the breathing. In particular a displacement 20 is illustrated on figure 7B. The displacement 20 specifies the difference between the positions of the bone 1 respectively illustrated in parts 7A and 7B. The displacement corresponds in this example in a translation of the target coordinate system TCS in a vertical direction with respect to the reference coordinate system RCS.

A second example of expected motions of the patient during surgery is the action on the bone 1 of an instrument used to achieve the surgery.

Parts 8A and 8B of figure 8 illustrate such an instrument-induced motion; each part illustrating the bone 1 and the tracker 10 at two different instants of this motion.

An instrument 30 is displaced to come close to contact with the bone 1 as illustrated in part 8A.

The instrument is further pushed against the bone 1 so that the bone 1 is displaced, as illustrated in part 8B. In particular, the bone 1 may pivot according to a tilt angle 21. The same tilt angle is experienced by the tracker 10 and eventually the target coordinate system TCS with respect to the reference coordinate system RCS.

### Breathing model

A breathing model may be used to add a set of expected movements of the tracker in a reference coordinate system.

A breathing model is a general description of a movement of the body due to breathing.

The model may be a non-patient-specific model, that is a generic model retrieved preoperatively. The breathing model may comprise parameters such as the age, the size and the mass of the body, the position of the body and its support constraints, the frequency and amplitude of breathing and also the shape of the breathing cycle.

In particular, during surgery, the patient is usually placed under artificial respiration using a respirator, imposing the frequency and the amplitude of breathing and the shape of the breathing cycle. A respirator dedicated to set those parameters may be connected to the control unit 101 in order to transmit them in real-time during surgery.

According to the values of the parameters provided, estimations of motions of a specific part of the body can be found using the breathing model, and especially a part or a bone of the thoracic cavity. A specific bone may also be a parameter of the breathing model.

The user may input, through the user interface 107, values for parameters comprised in the breathing model, the values corresponding to the current surgical operation. The control unit can then determine a set of expected motions of the bone due to breathing.

For instance, the translation motion and the rotation motion of a vertebra during a breathing cycle may be estimated from a breathing model. For a given bone of the patient, the breathing model provides a curve of a displacement of the bone over time in at least three degrees of freedom (such as three translations according to the directions of the reference coordinate system) but preferably six degrees of freedom for more accuracy (i.e. the three translations and three rotations around the directions of the reference coordinate system).

Those estimations of motions can be added to the set of expected motions and recorded in the memory of the system.

Alternatively, expected motions due to the breathing may be acquired intraoperatively. For example, said motions may be acquired through the tracking assembly attached to the patient. The acquisition may last at least one breathing cycle, and more preferably several breathing cycles to build a more accurate model. The acquisition may take place just after the attachment of the tracker and before any manipulation or intervention on the patient.

The curves of displacements initially determined thanks to the breathing model or acquired intraoperatively will be valid for the entire duration of the surgery, if the anesthesiologist does not change the parameters of the artificial respiration. However, if the frequency and amplitude of breathing are modified during surgery, curves of displacements may be updated or be recorded once again.

In the situation where frequency and amplitude of breathing are modified during surgery, it may be suggested via the user interface 107 to proceed to the determination of new expected motions.

### Bone/instrument interaction model

A bone/instrument interaction model may be used to determine expected movements of the tracker.

Such a model comprises a biomechanical description of the bone, due to the interaction with the neighbouring bones and tissues. The model comprises parameters such as the bone involved, the entry point - the point of the bone where the instrument comes into contact with the bone -, the force applied, the instrument, the category of instrument, or an instrument model. An instrument model describes the forces possibly exerted by an instrument as a function of its rigidity, its mass, its shape, its motion...

Other possible parameters of model include the force (magnitude, axis and direction) exerted on the bone by the instrument, the speed and acceleration of the instrument at the moment of impact, a motion of a part of the instrument in contact with the bone, such as the rotation of a drilling part on the distal end of the instrument, a rotation speed of a rotating part of the instrument.

Knowing that a bone sustains a force of an instrument at a point, a motion of the bone may be determined using the bone/instrument interaction model. Such a model enables to estimate a physical reaction of a bone to an instrument acting on the bone.

For a given bone of the patient and a given instrument exerting a given force, the bone/instrument interaction model provides an estimation of the displacement of the bone over time in at least three degrees of freedom (such as three translations according to the directions of the reference coordinate system) but preferably six degrees of freedom for more accuracy (the three translations and three rotations around the directions of the reference coordinate system).

The control unit 101 is connected to the localization system or the robotic system to receive in real-time the position and orientation of the instrument. Values of parameters of the bone/instrument interaction model are thus provided to the control unit 101, including for instance the point of contact with the bone, the trajectory (pose, speed, acceleration), the force exerted on the tip of the instrument (i.e. on the bone).

The control unit can then determine an expected motion of the bone.

When a surgical robotic system is used, a feedback from the surgical robotic system may provide input parameters to the bone/instrument interaction model. For instance, the force exerted on the bone by an instrument held by the surgical robotic system may be deduced from the measurements on the torque sensors embedded in the robotic arm.

The control unit is configured to receive a position and an orientation of the navigated instrument, and further to determine the set of expected motions in function of the position and the orientation of the navigated instrument.

The control unit is configured to receive one of the following parameters of the instrument: an entry point in the bone, an entry angle and a velocity, the control unit being configured to determine the set of expected motions in function of the one of these parameters.

### Determination of the entry point of an instrument into the bone

If an instrument is about to mechanically interact with a bone, expected movements of the bone may be greater in amplitude, speed and acceleration and also in a wider variety of directions.

Several ways may be used to determine the distance separating the instrument tip and the bone and also to determine the entry point of the instrument.

A first way to determine the distance and the entry point was previously described based on a planning or a virtual trajectory that a surgical instrument is intended to follow in the body of a patient.

A second way to determine the distance and the entry point is based on a local image segmentation of the body volume surrounding the bone in which the virtual trajectory is planned and the instrument navigated. Those images are acquired intraoperatively by the X-ray imaging system. As the instrument gets closer to the bone, the entry point may be identified on the acquired images.

A third way to determine the distance and the entry point is based on an input of the surgeon or user. If the surgeon estimates that the navigated instrument has indeed reached the entry point, the surgeon may input this information in the system, for example through the user interface 107. Then, the current position of the tip of the instrument is recorded as the position of the entry point.

### Identification of instrument for better definition of expected movements

Moreover, the instrument may present an identifier, for example a predetermined type of pattern for the tracking assembly attached to it, that allows for a better determination of the set of expected motions associated to the mechanical interaction between said instrument and the bone.

For example, if a pattern attached to an instrument indicates that it is a drilling bit, the set of expected motions will take into account the specifics of the mechanical interaction between the drilling bit and the bone, i.e. the rotational motion of the distal end of the drilling bit against the bone at a predetermined, high speed.

Moreover, and beyond motorized and/or manual movements of an instrument such as the rotation of a drill bit or mill head or a translation of a saw, the identification of the instrument may give the dimensions of said instrument and other parameters as well: width/diameter/form of the tip, material properties such as stiffness, in other words parameters that may have an impact of the bone/instrument mechanical interaction model.

The system for identifying an unexpected motion of a patient tracker attached to a patient during surgery may be implemented through the following method for identifying an unexpected motion of a patient tracker attached to a patient during surgery, the method comprising the steps of:
a. Coupling the patient tracker to a localization system,
b. Determining a set of expected motions of the patient tracker based on a set of expected motions of a bone of a patient during surgery,
c. Monitoring a motion of the patient tracker during surgery,
d. Determining a difference between the motion of the patient tracker and the set of expected motions of the patient tracker,
e. Emitting an alert of an unexpected motion when the difference exceeds a predetermined threshold.

The method may further comprise a step of: f) proposing to the user to add the unexpected motion to the set of expected motions and adding the unexpected motion to the set of expected motions when an approval is received from the user.

The method may further comprise a step of: g) modifying the model to consider the unexpected motion as an expected motion, when the user decides to add the unexpected motion to the set of expected motions.

Optionally, the set of expected motions of the bone may be produced based on a model.

The model may include a breathing model of the patient.

Optionally, the method further comprises a step of recording motions of the patient tracker during a breathing cycle intraoperatively, and adding the recorded motion to the set of expected motions.

The model may include a interaction bone/instrument model of the patient, the interaction bone/instrument model taking into account physical reactions of a bone to an instrument navigated acting on the bone.

The reception of the position and the orientation of the navigated instrument may be combined with the position and the orientation of the bone. A set of expected motions in function of the position and the orientation of the navigated instrument can be determined.

Other parameters of the instrument may also be received such as an entry position in the bone, an entry angle and a velocity. A set of expected motions in function of these parameters can then be determined.

The predetermined threshold may depend on a distance between a tip of a navigated instrument and a surgical target, for instance the predetermined threshold increases when the distance decreases.

Optionally, the navigated instrument is guided by a surgical robotic system, and possibly autonomously actuated by the surgical robotic system.

The method may further comprise a step of defining a reference coordinate system fixed with respect to an operating table, wherein the motion of the patient tracker and the set of expected motions of the patient tracker are expressed in the reference coordinate system.

Monitoring the motion of the patient tracker during surgery may comprise monitoring a position and orientation of a reference tracker located on the base of the surgical robotic system, the base being fixed with respect to the operating table, and calculating a motion of the patient tracker with respect to the position and orientation of the reference tracker.

Alternatively, monitoring the motion of the tracker during surgery comprises monitoring a position and orientation of a reference tracker located on an end-effector of a robotic arm of the surgical robotic system, calculating a position and orientation of the reference coordinate system based on a geometrical model of the robotic arm and calculating a motion of the patient tracker with respect to the reference coordinate system.

## Claims

1. System (100) for identifying an unexpected motion of a patient tracker (10) attached to a patient (2) during surgery, the system (100) comprising
a. a patient tracker (10)
b. a localization system (103) coupled to the patient tracker (10),
c. a memory (102) in which a set of expected motions of the patient tracker is recorded,
d. a control unit (101) connected to the memory (102) and the localization system (103), the control unit (101) being configured to
monitor a motion of the patient tracker (10) during surgery, determine a difference between the motion of the patient tracker (10) and the set of expected motions of the patient tracker (10), and
compare the difference to a predetermined threshold,
e. an alert module (105) connected to the control unit (101), the alert module (101) being configured to emit an alert of an unexpected motion, when the difference exceeds the predetermined threshold.

2. System (100) according to claim 1, further comprising a user interface (107) configured to
display a proposition to the user to add the unexpected motion to the set of expected motions and
receive a refusal or an approval from the user,
the control unit (101) being configured to add the unexpected motion to the set of expected motions when an approval is received from the user.

3. System (100) according to claim 1, wherein the set of expected motions is produced based on a model.

4. System (100) according to claim 3, wherein the control unit (101) is further configured to modify the model so as to consider the unexpected motion as an expected motion, when an approval is received from the user.

5. System (100) according to any of claims 3 to 4, wherein the model includes a breathing model of the patient.

6. System (100) according to any preceding claim, wherein the control unit (101) is configured to record a reference motion of the patient tracker (10) during a breathing cycle intraoperatively, and add the reference motion to the set of expected motions.

7. System (100) according to any preceding claim, wherein the control unit (101) is configured to receive a position and an orientation of a navigated instrument (30), the control unit (101) being configured to determine the set of expected motions in function of the position and the orientation of the navigated instrument (30).

8. System (100) according to claim 7, wherein the control unit (101) is configured to receive at least one of the following parameters of the instrument (30): an entry point in the bone, an entry angle and a velocity, the control unit (101) being configured to determine the set of expected motions in function of said at least one parameter.

9. System (100) according to any of claims 7 and 8, wherein the control unit (101) is configured to modify the predetermined threshold depending on a distance between a tip of the navigated instrument and a surgical target.

10. System (100) according to claim, 9, wherein the control unit (101) is configured to increase the predetermined threshold when the distance decreases.

11. System (100) according to any of claims 7 to 10, further comprising a surgical robotic system (70) configured to guide the instrument (30).

12. System according to any of claims 7 to 11, further comprising a surgical robotic system (70) configured to autonomously actuate the instrument (30).

13. System (100) according to any of claims 1 to 12, wherein the control unit (101) is configured to define a reference coordinate system fixed with respect to an operating room, the control unit (101) being configured to express the motion of the patient tracker (10) and the set of expected motions of the patient tracker (10) in the reference coordinate system.

14. System (100) according to claim 13 in combination with claim 11 or claim 12, wherein the control unit (101) is configured to receive from the localization system (103) localization data relating to a position and an orientation of a reference tracker (40) located on a base of the surgical robotic system, the base being fixed with respect to the operating room, the control unit (101) being configured to calculate a motion of the patient tracker (10) with respect to the position and orientation of the reference tracker (40).

15. System (100) according to claim 13 in combination with claim 11 or claim 12, wherein the control unit (101) is configured to receive from the localization system (103) localization data relating to a position and an orientation of a reference tracker (40) located on an end-effector of a robotic arm of the surgical robotic system (70), the control unit (101) being configured to calculate a position and orientation of the reference coordinate system based on a geometrical model of the robotic arm and calculate a motion of the patient tracker (10) with respect to the reference coordinate system.
